# EUROPEAN PATENT SPECIFICATION

(11) **EP 2 710 996 B1**
(45) Date of publication and mention of the grant of the patent: **17.08.2022**
(21) Application number: 13198908.9
(22) Date of filing: 05.12.2008
(51) Int. Cl.: A61K 8/19, A61K 8/49, A61Q 1/02, A61Q 17/04, A61Q 19/08

(54) **Sunscreen compositions comprising colour pigments**
Sonnenschutzzusammensetzungen mit Farbpigmenten
Compositions d'écran solaire comprenant des pigments de couleur

(30) Priority: 14.12.2007 EP 07123286; 06.05.2008 EP 08155725
(43) Date of publication of application: 26.03.2014
(62) Divisional of application: 08863197.3
(73) Proprietor: BASF SE, 67056 Ludwigshafen am Rhein (DE)
(72) Inventor: Ehlis, Thomas, 79100 Freiburg (DE); Sohn, Myriam, 68300 Saint-Louis (FR)
(74) Representative: BASF IP Association

(56) References cited:
- EP-A1- 1 709 954
- EP-A1- 1 782 792
- WO-A1-2004/098546
- WO-A1-2006/034991
- WO-A2-2004/085412
- WO-A2-2007/071584
- DE-A1- 10 035 512
- Ciba Specialty Chemicals: "Ciba Tinosorb M - Technical Data Sheet", , 26 May 2004 (2004-05-26), pages 1-2, XP002591733, Retrieved from the Internet: URL:http://www.imminst.org/forum/index.php ?s=8325ff68f2c386b48dc4877968780287&app=co re&module=attach&section=attach&attach_rel _module=post&attach_id=7932 [retrieved on 2010-07-12]
- "Use of amino hydroxy benzophenone derivatives in sunscreen preparations", IP.COM JOURNAL, IP.COM INC., WEST HENRIETTA, NY, US, 24 August 2006 (2006-08-24), XP013115567, ISSN: 1533-0001

## Description

The present invention relates to cosmetic compositions comprising micronized and/or soluble UV absorbers and one or more color pigments.

It has long been known that prolonged exposure to that UV radiation which reaches the surface of the earth can lead to the formation of erythemas or light dermatoses, as well as to an increased incidence of skin cancers or accelerated skin aging.

Various sunscreen formulations have been proposed which include actives which are intended to counteract UV radiation, thereby inhibiting the said undesired effects on the skin. A great number of compounds has been proposed for the use as UV protectants in sunscreen formulations, especially soluble organic UV absorbers and also insoluble micronized organic and inorganic compounds, which become more and more common in sunscreen formulations.

Micronised, insoluble organic UV absorbers, when used in sunscreen formulations, provide excellent UV protection and have a high SPF rating. Moreover, micronised, insoluble organic UV absorbers show no tendency, under the influence of light, to generate radicals which could damage or sensitise human skin.

'Use of amino hydroxy benzophenone derivatives in sunscreen preparations ', IP.com Journal, IP.com Inc. West Henrietta, NY, US, 24 August 2006, XP013115567, ISSN: 1533-0001 discloses the use of specific micronized UV absorbers (amino hydroxyl benzophenone derivatives for protecting human hair and skin against UV radiation and skin aging and preventing tanning.

WO 2007/071584 A2 discloses the use of an aqueous dispersion comprising a micronized sparingly soluble organic compound against UV radiation and skin aging and preventing tanning and cosmetic or dermatological compositions comprising these dyes.

Unfortunately, due to light scattering sun screen compostitions comprising organic and/or inorganic particulate filters show an articulate whitening aspect when applied on the skin.

The whitening aspect increases with higher concentration of the corresponding UV filters and will therefore limit their required quantity.

Furthermore, soluble and particulate UV filters which have absorption maximum in the UV-A region are mostly not colourless since these substances will absorb also light in the visible range of the spectrum. Due to the particular colour of these UV-A filters the resulting cosmetic sun screen formulations will be coloured. The hue of such formulations (in most cases yellowish) is considered as unnatural since it derivates significantly from the natural skin hue.

Furthermore, it is known that some UV filters show significant fluorescence. Artificial illuminations with a high UV ratio therefore generate very unnatural skin tones.

Surprisingly it has been found that the combination of particulate or soluble organic UV filters with one or more colour pigments provides cosmetic sunscreen formulations which have a hue that corresponds to the natural skin tone and is therefore not considered as unnatural.

Therefore the present invention relates to non-therapeutic use of a cosmetic formulation comprising
(a) particulates having an absorption in the range of 400 to 800nm selected from inorganic colour pigments selected from iron (III) oxide
   ; and
(b)
   particulate organic UV-filter of formula

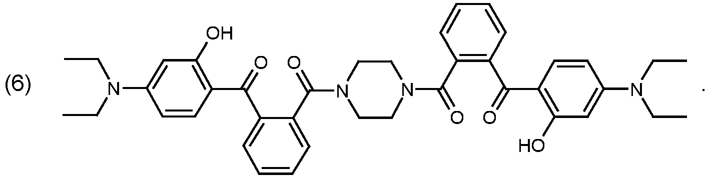

Of specific interest are the pigments/colours listed in Table 1 below:

| Table 1a: Examples of piqments/colours (component (b)) | | |
|---|---|---|
| Color index number | Official and common name | CAS reqistry number |
| 10006 | Piqment Green 8 | 16143-80-9 |
| 10020 | Acid Green 1 | 19381-50-1 |
| 10316 (2) | Ext. D & C Yellow No. 7; Acid Yellow 1;Naphthol Yellow S | 846-70-8 |
| 11680 | Piqment Yellow 1 | 2512-29-0 |
| 11710 | Piqment Yellow 3 | 6486-23-3 |
| 11725 | Pigment Orange 1 | 6371-96-6 |
| 11920 | Solvent Oranqe 1 | 2051-85-6 |
| 12010 | Solvent Red 3 | 6535-42-8 |
| 12085 (2) | D & C Red No. 36; Piqment Red 4; Flaming Red | 2814-77-9 |
| 12120 | Pigment Red 3 | 2425-85-6 |
| 12150 | Solvent Red 1 | 1229-55-6 |
| 12370 | Piqment Red 112 | 6535-46-2 |
| 12420 | Piqment Red 7 | 6471-51-8 |
| 12480 | Piqment Brown | 6410-40-8 |
| 12490 | Pigment Red 5 | 6410-41-9 |
| 12700 | Disperse Yellow 16 | 4314-14-1 |
| 13015 | E 105; Acid Yellow 9 | 2706-28-7 |
| 14270 | E 103; Acid Orange 6 | 547-57-9 |
| 14700 | FD & C Red No. 4; Food Red 1; Ponceau SX | 4548-53-2 |
| 14720 | E 122; Acid Red 14; Food Red 3; Azorubine; Carmoisine | 3567-69-9 |
| 14815 | E 125; Food Red 2 | 3257-28-1 |
| 15510 (2) | D&C Oranqe No. 4; Acid Orange 7; Orange II | 633-96-5 |
| 15525 | Piqment Red 68 | 5850-80-6 |
| 15580 | Piqment Red 51 | 227-459-1 |
| 15620 | Acid Red 88 | 1658-56-6 |
| 15630 | Piqment Red 49 | 1248-18-6 |
| 15800: 1 | D&C Red No. 31; Piqment Red 64:1; Brilliant Lake Red R | 6371-76-2 |
| 15850 | D&C Red No. 6; Piqment Red 57; Lithol Rubin B | 5858-81-1 |
| 15850:1 (2) | D&C Red No. 7; Pigment Red 57:1; Lithol Rubin B Ca | 5281-04-9 |
| 15865 (2) | Piqment Red 48 | 3564-21-4 |
| 15880: 1 | D&C Red No. 34; Pigment Red 63:1; Deep Maroon; Fanchon Maroon; Lake Bordeaux B | 6417-83-0 |
| 15980 | E 111; Food Orange 2 | 2347-72-0 |
| 15985 (2) | FD&C Yellow No. 6; E 110; Food Yellow 3; Sunset Yellow; Sunset Yellow FCF; Orange Yellow S | 2783-94-0 |
| 16035 | FD&C Red No. 40; Food Red 17; Allura Red; Allura Red AC | 25956-17-6 |
| 16230 | Acid Oranqe 10 | 1936-15-8 |
| 16255 (2) | E 124; Acid Red 18; Food Red 7; Ponceau 4R; Cochineal Red A | 2611-82-7 |
| 16290 | E 126; Acid Red 41 | 5850-44-2 |
| 17200 (2) | D&C Red No. 33; Acid Red 33; Acid Fuchsine | 3567-66-6 |
| 18050 | Acid Red 1 | 3734-67-6 |
| 18130 | Acid Red 155 | 10236-37-0 |
| 18690 | Acid Yellow 121 | 5601-29-6 |
| 18736 | Acid Red 180 | 6408-26-0 |
| 18820 | Acid Yellow 11 | 6359-82-6 |
| 18965 | Acid Yellow 17 | 6359-98-4 |
| 19140 (2) | FD&C Yellow No. 5; E 102; Acid Yellow 23; Tartrazine | 1934-21-0 |
| 20040 | Piqment Yellow 16 | 5979-28-2 |
| 20170 | D&C Brown No. 1; Acid Orange 24; Resorcin Brown | 1320-07-6 |
| 20470 | Noir W 699 | 1064-48-8 |
| 21100 | Piqment Yellow 13 | 5102-83-0 |
| 21108 | Pigment Yellow 83 | 5567-15-7 |
| 21230 | Solvent Yellow 29 | 6706-82-7 |
| 24790 | Acid Red 163 | 13421-53-9 |
| 26100 | D&C Red No. 17; Solvent Red 23; Sudan III; Toney Red | 85-86-9 |
| 27290 (2) | Acid Red 73 | 5413-75-2 |
| 27755 | E152 ; Food Black 2 | 2118-39-0 |
| 28440 | E 151; Brilliant Black 1; Food Black 1; Brilliant Black BN; Black PN | 2519-30-4 |
| 40215 | Direct Oranqe 34 | 1325-54-8 |
| 40800 | Beta Carotene (synthetic) ; Food Orange 5 | 7235-40-7 |
| 40820 | E 160e; Food Orange 6; β-Apo-8' Carotenal (C 30) | 1107-26-2 |
| 40825 | E 160f; Food Orange 7; Ethyl Ester of β-Aρο-8' Carotenic Acid (C 30) | 1109-11-1 |
| 40850 | Canthaxanthin; E 161g; Food Orange 8 | 514-78-3 |
| 42045 | Acid Blue 1 | 129-17-9 |
| 42051 (2) | E 131; Acid Blue 3; Patent Blue V | 3536-49-0 |
| 42053 | FD&C Green No. 3; Food Green 3; Fast Green FCF | 2353-45-9 |
| 42080 | Acid Blue 7 | 3486-30-4 |
| 42090 | FD&C Blue No. 1; Acid Blue 9 (Sodium salt) ; Food Blue 2; Brilliant Blue FCF; Patent Blue AC | 3844-45-9 |
| | FD&C Blue No. 4; Acid Blue 9 (Ammonium salt) ; Alphazurine FG; Erioglaucine | 6371-85-3 |
| 42100 | Acid Green 9 | 4857-81-2 |
| 42170 | Acid Green 22 | 5863-51-4 |
| 42510 | Basic Violet 14 | 632-99-5 |
| 42520 | Basic Violet 2 | 3248-91-7 |
| 42735 | Acid Blue 104 | 6505-30-2 |
| 44045 | Basic Blue 26 | 2580-56-5 |
| 44090 | E142 ; Acid Green 50; Green S | 3087-16-9 |
| 45100 | Acid Red 52 | 3520-42-1 |
| 45190 | Acid Violet 9 | 6252-76-2 |
| 45220 | Acid Red 50 | 5873-16-5 |
| 45350 | D&C Yellow No. 8; Acid Yellow 73; Uranine | 518-47-8 |
| 45350:1 | D&C Yellow No. 7; Solvent Yellow 94; Fluorescein | 2321-07-5 |
| 45370 (2) | Acid Oranqe 11 | |
| 45370:1 | D&C Oranqe No. 5; Solvent Red 72; Dibromofluorescein | 596-03-2 |
| 45380 (2) | D&C Red No. 22; Acid Red 87; Eosin Y | 17372-87-1 |
| 45380:2 | D&C Red No. 21; Solvent Red 43; Tetrabromofluorescein | 15086-94-8 |
| 45396 | Solvent Oranqe 16 | 24545-86-6 |
| 45405 | Acid Red 98 | 6441-77-6 |
| 45410 (2) | D&C Red No. 28; Acid Red 92; Phloxine B | 18472-87-2 |
| 45410:1 | D&C Red No. 27; Solvent Red 48; Tetrabromotetrachloro-; Fluorescein | 13473-26-2 |
| 45425 | D&C Red No. 11; Acid Red 95; Erythrosine Yellowish Na | 33239-19-9 |
| 45425:1 | D&C Oranqe No. 10; Solvent Red 73; Diiodofluorescein | 38577-97-8 |
| 45430 (2) | E 127; FD&C Red No. 3; Acid Red 51; Erythrosine; Food Red 14 | 16423-68-0 |
| 47000 | D&C Yellow No. 11; Solvent Yellow 33; Quinoline Yellow SS | 8003-22-3 |
| 47005 | D&C Yellow No. 10; E104; Acid Yellow; Food Yellow 13; Quinoline Yellow; Canary Yellow | 8004-92-0 |
| 50325 | Acid Violet 50 | 229-951-1 |
| 50420 | Acid Black 2 | 8005-03-6 |
| 51319 | Piqment Violet 23 | 6358-30-1 |
| 58000 | Piqment Red 83; Alizarin | 72-48-0 |
| 59040 | D&C Green No. 8; Solvent Green 7; Pyranine Concentrated | 6358-69-6 |
| 60724 | Disperse Violet 27 | 19286-75-0 |
| 60725 | D&C Violet No. 2; Solvent Violet 13; Alizurol Purple SS | 81-48-1 |
| 60730 | Ext D&C Violet No. 2; Acid Violet 43 | 4430-18-6 |
| 61565 | D&C Green No. 6; Solvent Green 3; Quinizarin Green SS | 128-80-3 |
| 61570 | D&C Green No. 5; Acid Green 25 | 4403-90-1 |
| 61585 | Acid Blue 80 | 4474-24-2 |
| 62045 | Acid Blue 65 | 4368-56-3 |
| 69800 | E 130; Vat Blue 4 | 81-77-6 |
| 69825 | D&C Blue No. 9; Vat Blue 6; Indanthrene Blue; Carbanthrene Blue | 130-20-1 |
| 71105 | Vat Oranqe 7; Pigment Orange 43 | 4424-06-0 |
| 73000 | Vat Blue 1; Indiqo | 482-89-3 |
| 73015 | FD&C Blue No. 2; E 132; Food Blue 1; Indigotine; Indigo Carmine | 860-22-0 |
| 73360 | D&C Red No. 30; Vat Red 1; Helindone Pink CN | 2379-79-0 |
| 73385 | Vat Violet 2 | 5462-29-3 |
| 73900 | Piqment Violet 19 | 1047-16-1 |
| 73915 | Pigment Red 122 | 16043-40-6 |
| 74100 | Piqment Blue 16 | 547-93-6 |
| 74160 | Piqment Blue 15 | 147-14-8 |
| 74180 | Direct Blue 86 | 1330-38-7 |
| 74260 | Piqment Green 7 | 1328-53-6 |
| 75100 | Natural Yellow 6 | 27876-94-4 |
| 75120 | Annatto extract; E 160b; Natural Orange 4 | 1393-63-1 |
| 75125 | E 160d; I ycopene | 502-65-8 |
| 75130 | Beta Carotene (Natural) ; E 160a; Natural Browns 5; Natural Yellow 26 | 7235-40-7 |
| 75135 | E 161d; Natural Yellow 27; Rubixanthin | 3763-55-1 |
| 75170 | Guanine; Natural White 1 | 73-40-5 |
| 75300 | E 100; Natural Yellow 3; Turmeric Yellow; Curcumin; Indian Saffron | 458-37-7 |
| 75470 | Cochineal extract; E120; Natural Red 4; Cochineal; Carmine; | 1343-78-8 |
| | Carminic Acid ............ | 1390-65-4 |
| | | 1260-17-9 |
| 75480 | Henna | 83-72-7 |
| 75810 | E 140 & E 141; Chlorophyll; Copper complexes of chlorophyll and chlorophyllins; Potassium sodium copper; Chlorophyllin; Natural Green 3 | 11006-34-1 |
| 77000 | Aluminum Powder; E 173; Piqment Metal 1; Aluminum | 4729-90-5 |
| 77002 | Alumina; Pigment White 24 | 1332-73-6 |
| 77004 | Piqment White 19; Bentonite | 1302-78-9 |
| 77007 | Ultramarine Blue; Pigment Blue 29 | 1317-97-1 |
| | Ultramarine Red | 1345-00-2 |
| | Ultramarine Violet; Pigment Violet 15 | 12769-96-9 |
| 77013 | Ultramarine Green; Piqment Green 24 | |
| 77015 | Piqment Red 101; Pigment Red 102 | |
| 77019 | Mica; Pigment White 20 | 12001-26-2 |
| | Talc; Pigment white 26 | 14807-96-9 |
| 77120 | Piqment White 21; Pigment White 22 | 7727-43-7 |
| 77163 | Bismuth oxychloride; Pigment White 14 | 7787-59-9 |
| 77220 | Calcium carbonate; E 170; Piqment White 18 | 471-34-1 |
| 77231 | Piqment White 25; Calcium Sulfate | 7778-18-9 |
| 77288 | Chromium Oxide Greens; Piqment Green 17 | 1308-38-4 |
| 77289 | Chromium hydroxide Green; Pigment Green 18; Guignet Green; Veridian | 12001-99-9 |
| 77346 | Cobalt Blue; Pigment Blue 28 | 1345-16-0 |
| 77400 77400 | Bronze powder; Copper powder; Pigment Metal 2 | 7440-50-8 |
| 77480 | E 175; Gold; Piqment Metal 3 | 7740-57-5 |
| 77489 | E 172; Ferrous oxide | 1345-25-1 |
| 77491 | Ferric oxide; E 172; Pigments Red 101 & 102; Pigments Brown 6 & 7 | 1309-37-1 |
| 77492 | Hydrated Ferric oxide; E 172; Pigments Yellow 42 & 43; Pigments Brown 6 & 7 | 20344-49-4 |
| 77499 | Ferrous Ferric oxide; E 172; Pigment Black 11; Pigments Brown 6 & 7 | 1317-61-9 |
| 77510 77520 | Ferric ferrocyanide; Pigment Blue 27; Prussian Blue | 14038-43-8 |
| 77713 | Maqnesium Carbonate; Pigment White 18 | 546-93-0 |
| 77742 | Manganese Violet; Pigment Violet 16 | 10101-66-3 |
| 77745 | Manqanous Phosphate | 10124-54-6 |
| 77820 | Silver; E 174 | 7440-22-4 |
| 77891 | Titanium Dioxide; E 171; Piqment White 6 | 13463-67-7 |
| 77947 | Zinc Oxide; Piqment White 4 | 1317-13-2 |
| | Acid red 195 | 12220-24-5 |
| | Aluminum distearate | 300-92-5 |
| | Anthocyanins; E 163 | 11029-12-2 |
| | Beetroot red; E 162 | 89957-89-1 |
| | | 7659-95-2 |
| | Bismuth citrate | |
| | Bromocresol green | 76-60-8 |
| | Bromothymol blue | 76-59-5 |
| | Calcium stearate | 1592-23-0 |
| | Capsanthin, Capsorubin; E 160c; Paprika extract | 465-42-9 |
| | E 150; Natural Brown 10; Caramel | 8028-89-5 |
| | Ferric ammonium ferrocyanide | 25869-00-5 |
| | E 101 ; Lactoflavin; Riboflavin | 83-88-5 |
| | Lead acetate | 301-04-2 |
| | Maqnesium Stearate | 557-04-0 |
| | Zinc Stearate | 557-05-1 |

| Table 1b: Structure of the Lakes: | | | |
|---|---|---|---|
| Lakes (FDA name) | CI No | name | structure of corresponding dye |
| D&C Red #6 Ba Lake | 15850 | Pigment Red 57 Sodium Salt | 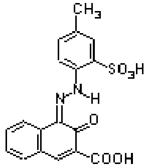 |
| D&C Red #7 | 15850:1 | Pigment Red 57.1 Calcium Salt | 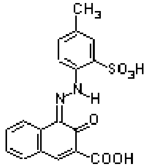 |
| D&C Red #21 AI Lake | 45380:2 | Solvent Red 43 | 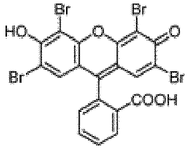 |
| D&C Red #22 Al Lake | 45380 | Acid Red 87 | 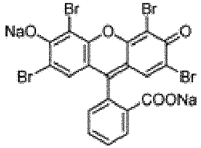 |
| D&C Red #27 Al Lake | 45410:1 | Solvent Red 48 | 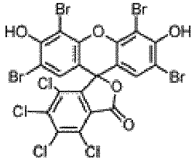 |
| D&C Red #28 Al Lake | 45410 | Acid Red 92 | 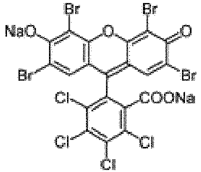 |
| D&C Red #30 Al & Talk Lake | 73360 | Vat Red 1 = Pigment Red 181 | 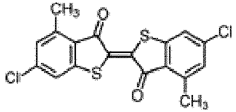 |
| D&C Red #33 Al & Zr Lake | 17200 | Acid Red 33 | 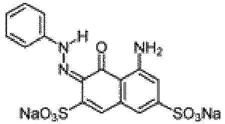 |
| D&C Yellow #10 Al Lake | 47005 | Food Yellow 13 | 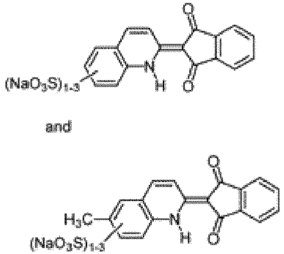 |
| FD&C Yellow # 5 Al Lake | 19140 | Acid Yellow 23 Na Salt | 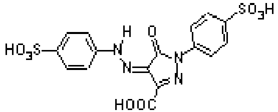 |
| FD&C Yellow #6 Al Lake | 15985 | Food Yellow 3 Na Salt | 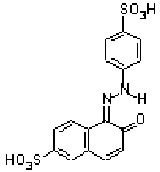 |
| FD&C Blue #1 Al Lake | 42090 | Acid blue 9 | |

| Table 1c: Calisha colour pigments (Ciba): | |
|---|---|
| Treated Pearls | |
| Name | INCI Name |
| Calisha Romance Silver Pearl OD | Mica and Titanium Dioxide and Cetyl Dimethicone |
| Calisha Romance Glamour Violet OD | Mica and Titanium Dioxide and Cetyl Dimethicone |
| Calisha Romance Glamour Blue Pearl OD | Mica and Titanium Dioxide and Cetyl Dimethicone |
| Calisha Romance Glamour Green OD | Mica and Titanium Dioxide and Cetyl Dimethicone |
| Calisha Romance Gold Pearl OD | Mica and Titanium Dioxide and Red Iron Oxide and Cetyl Dimethicone |
| Calisha Romance Bronze Pearl OD | Mica and Red Iron Oxide and Cetyl Dimethicone |
| Calisha Romance Orange Pearl OD | Mica and Red Iron Oxide and Cetyl Dimethicone |
| Calisha Romance Red Pearl OD | Mica and Red Iron Oxide and Cetyl Dimethicone |
| Calisha Contemporary Starlight Pearl OD | Mica and Titanium Dioxide and Cetyl Dimethicone |
| Calisha Contemporary Glamour Blue Pearl OD | Mica and Titanium Dioxide and Cetyl Dimethicone |
| Calisha Contemporary Glamour Gold Pearl OD | Mica and Titanium Dioxide and Cetyl Dimethicone |
| Calisha Contemporary Glamour Violet OD | Mica and Titanium Dioxide and Cetyl Dimethicone |
| Calisha Contemporary Claret Pearl OD | Mica and Red Iron Oxide and Titanium Dioxide and Cetyl Dimethicone |
| Calisha Contemporary Sienna Pearl OD | Mica and Red Iron Oxide and Titanium Dioxide and Cetyl Dimethicone |
| Calisha Contemporary Black Pearl OD | Mica and Titanium Dioxide and Black Iron Oxide and Cetyl Dimethicone |
| Calisha Contemporary Sable Pearl OD | Mica and Titanium Dioxide and and Red Iron Oxide Carmine and Cetyl Dimethicone |
| Calisha Contemporary Green Pearl OD | Mica and Titanium Dioxide and Red Iron Oxide and Ferric Ferrocyanide and Cetyl Dimethicone |
| Calisha Contemporary Silver Pearl OD | Mica and Titanium Dioxide and Cetyl Dimethicone |
| Calisha Contemporary Glamour Green OD | Mica and Titanium Dioxide and Tin Dioxide and Cetyl Dimethicone |
| Calisha Contemporary Gold Pearl OD | Mica and Titanium Dioxide and Red Iron Oxide and Cetyl Dimethicone |
| Calisha Contemporary Bronze Pearl OD | Mica and Red Iron Oxide and Cetyl Dimethicone |
| Calisha Contemporary Rose Red Pearl OD | Mica and Red Iron Oxide and Cetyl Dimethicone |
| Calisha Art Deco Silver Pearl OD | Mica and Titanium Dioxide and Cetyl Dimethicone |
| Calisha Art Deco Glamour Violet OD | Mica and Titanium Dioxide and Cetyl Dimethicone |
| Calisha Art Deco Glamour Blue Pearl OD | Mica and Titanium Dioxide and Cetyl Dimethicone |
| Calisha Art Deco Glamour Green OD | Mica and Titanium Dioxide and Cetyl Dimethicone |
| Calisha Art Deco Gold Pearl OD | Mica and Red Iron Oxide and Titanium Dioxide and Cetyl Dimethicone |
| Calisha Art Deco Bronze Pearl OD | Mica and Red Iron Oxide and Cetyl Dimethicone |
| Calisha Art Deco Orange Pearl OD | Mica and Red Iron Oxide and Cetyl Dimethicone |
| Calisha Art Deco Red Pearl OD | Mica and Red Iron Oxide and Cetyl Dimethicone |

| Untreated Pearls | |
|---|---|
| Calisha Romance Silver Pearl CL | Mica and Titanium Dioxide |
| Calisha Romance Glamour Violet CL | Mica and Titanium Dioxide |
| Calisha Romance Glamour Blue Pearl CL | Mica and Titanium Dioxide |
| Calisha Romance Glamour Green CL | Mica and Titanium Dioxide |
| Calisha Romance Gold Pearl CL | Mica and Titanium Dioxide and Red Iron Oxide |
| Calisha Romance Bronze Pearl CL | Mica and Red Iron Oxide |
| Calisha Romance Orange Pearl CL | Mica and Red Iron Oxide |
| Calisha Romance Red Pearl CL | Mica and Red Iron Oxide |
| Calisha Contemporary Starlight Pearl CL | Mica and Titanium Dioxide |
| Calisha Contemporary Glamour Blue Pearl CL | Mica and Titanium Dioxide |
| Calisha Contemporary Glamour Gold Pearl CL | Mica and Titanium Dioxide |
| Calisha Contemporary Glamour Violet CL | Mica and Titanium Dioxide |
| Calisha Contemporary Claret Pearl CL | Mica and Red Iron Oxide and Titanium Dioxide |
| Calisha Contemporary Sienna Pearl CL | Mica and Red Iron Oxide and Titanium Dioxide |
| Calisha Contemporary Black Pearl CL | Mica and Titanium Dioxide and Black Iron Oxide |
| Calisha Contemporary Sable Pearl CL | Mica and Titanium Dioxide and Red Iron Oxide and Carmine |
| Calisha Contemporary Green Pearl CL | Mica and Titanium Dioxide Red Iron Oxide Ferric Ferrocyanide |
| Calisha Contemporary Silver Pearl CL | Mica and Titanium Dioxide |
| Calisha Contemporary Glamour Green CL | Mica and Titanium Dioxide and Tin Dioxide |
| Calisha Contemporary Gold Pearl CL | Mica and Titanium Dioxide and Red Iron Oxide |
| Calisha Contemporary Bronze Pearl CL | Mica and Red Iron Oxide |
| Calisha Contemporary Rose Red Pearl CL | Mica and Red Iron Oxide |
| Calisha Art Deco Silver Pearl CL | Mica and Titanium Dioxide |
| Calisha Art Deco Glamour Violet CL | Mica and Titanium Dioxide |
| Calisha Art Deco Glamour Blue Pearl CL | Mica and Titanium Dioxide |
| Calisha Art Deco Glamour Green Pearl CL | Mica and Titanium Dioxide |
| Calisha Art Deco Gold Pearl CL | Mica and Red Iron Oxide and Titanium Dioxide |
| Calisha Art Deco Bronze Pearl CL | Mica and Red Iron Oxide |
| Calisha Art Deco Orange Pearl CL | Mica and Red Iron Oxide |
| Calisha Art Deco Red Pearl CL | Mica and Red Iron Oxide |

| Inorganic Pigments | |
|---|---|
| Calisha Contemporary Salmon Red Oxide | Red Iron Oxide |
| Calisha Contemporary Medium Red Oxide | Red Iron Oxide |
| Calisha Contemporary Deep Red Oxide | Red Iron Oxide |
| Calisha Contemporary Black Oxide | Black Iron Oxide |
| Calisha Contemporary Jet Black Oxide | Black Iron Oxide |
| Calisha Contemporary Sun Yellow Oxide | Yellow Iron Oxide |
| Calisha Contemporary Yellow Oxide | Yellow Iron Oxide |
| Calisha Contemporary Ultramarine Blue | Ultramarine Blue |
| Calisha Contemporary Ultramarine Violet | Ultramarine Violet |
| Calisha Art Deco Hydroxide Chrome Green | Chromium Hydroxide Green |
| Calisha Art Deco Chrome Oxide Green | Chromium Oxide Greens |

| Lakes | |
|---|---|
| Calisha Art Deco Blue 1 Lake | FD&C Blue No. 1 Al Lake |
| Calisha Art Deco Red 21 Lake | D&C Red No. 21 Al Lake |
| Calisha Art Deco Red 27 Lake | D&C Red No. 27 Al Lake |
| Calisha Art Deco Red 30 AL. Lake | D&C Red No. 30 Al Lake |
| Calisha Art Deco Red 30 Talc Lake | D&C Red No. 30 Talc Lake |
| Calisha Art Deco Red 33 Lake | D&C red No. 33 Al Lake |
| Calisha Art Deco Red 40 AL. Lake | FD&C Red No. 40 Al lake |
| Calisha Art Deco Red 6 Lake HD | D&C Red No. 6 Ba Lake |
| Calisha Art Deco Red 7 Lake HD | D&C Red No. 7 Ca lake |
| Calisha Art Deco Yellow 5 Lake HD | FD&C Yellow No. 5 Al Lake |
| Calisha Art Deco Yellow 6 AL. Lake | FD&C Yellow No. 6 Al lake |

The interference pigments as used in the present invention are platelet particulates. The platelet particulates preferably have a thickness of no more than about 5 micrometers, more preferably no more than about 2 micrometers, still more preferably no more than about 1 micrometer. The platelet particulates preferably have a thickness of at least about 0.02 micrometers, more preferably at least about 0.05 micrometers, even more preferably at least about 0.1 micrometers, and still more preferably at least about 0.2 micrometers.

The particle size determines the opacity and luster. The particle size is determined by measuring the diameter thickness of the particulate material. The term "diameter" as used herein, means the largest distance across the major axis of the particulate material. Diameter can be determined by any suitable method known in the art, such as particle size analyzer Mastersizer 2000 manufactured by Malvern Instruments. The interference pigments preferably have an average diameter not greater than about 200 micrometers, more preferably not greater than 150 micrometers. The interference pigments preferably have a diameter of at least about 0.1 micrometer, more preferably at least about 1.0 micrometer, even more preferably at least about 2.0 micrometer, and still more preferably at least about 5.0 micrometer.

The interference pigments comprise a multilayer structure. The center of the particulates is a flat substrate with a refractive index (RI) normally below 1.8. A wide variety of particle substrates are useful herein. Nonlimiting examples are natural mica, synthetic mica, graphite, talc, kaolin, alumina flake, bismuth oxychloride, silica flake, glass flake, ceramics, titanium dioxide, CaSO₄, CaCO₃, BaSO₄, borosilicate and mixtures thereof, preferably mica, silica and alumina flakes.

A layer of thin film or a multiple layer of thin films are coated on the surface of a substrate described above. The thin films are made of highly refractive materials. The refractive index of these materials is normally above 1.8.

A wide variety of thin films are useful herein. Nonlimiting examples are TiO₂, Fe₂O₃, SnO₂, Cr₂O₃, ZnO, ZnS, SnO, ZrO₂, CaF₂, Al₂O₃, BiOCI, and mixtures thereof or in the form of separate layers, preferably TiO₂, Fe₂O₃, Cr₂O₃, and SnO₂.

For the multiple layer structures, the thin films can be consisted of all high refractive index materials or alternation of thin films with high and low RI materials with the high RI film as the top layer.

The interference pigment color is a function of the thickness of thin film, the thickness for a specific color may be different for different materials. For TiO₂, a layer of 40nm to 60nm or a whole number multiple thereof gives silver color, 60nm to 80nm yellow color, 80nm to 100nm red color, 100nm to 130nm blue color, 130nm to 160nm green color.

In addition to the interference color, other transparent absorption pigments can be precipitated on top of or simultaneously with the TiO₂ layer. Common materials are red or black iron oxide, ferric ferrocyanide, chromium oxide or carmine. It is found that the color of the interference pigment in addition to its brightness has a significant influence on human perception of skin tone. In general, preferred colors are silver, gold, red, green and mixtures thereof.

Nonlimiting examples of the interference pigments useful herein include those supplied by Persperse, Inc. under the trade name PRESTIGE.RTM., FLONAC.RTM.; supplied by EMD Chemicals, Inc. under the trade name TIMIRON.RTM., COLORONA.RTM., DICHRO-NA.RTM. and XIRONA.RTM.; supplied by Engelhard Co. under the trade name FLAMENCO.RTM., TIMICA.RTM., DUOCHROME.RTM.; and supplied by Ciba under the trade name CALISHA.RTM.

Although there are no critical limitations to the sizes of the pigment particles, preferably pigment particles having a size from about 0.01 to about 500 micrometers, more preferably having a size of about 1 to about 200 micrometers, most preferably having a size from about 5 to about 150 micrometers are modified with the silicone compounds according to formula 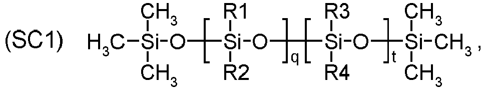 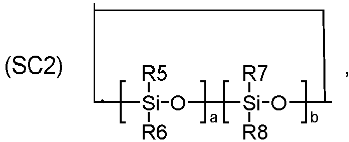 and 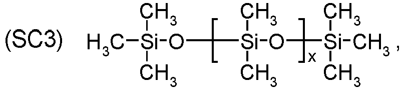 wherein
- R1: is C1 to C22 branched or straight chain alkyl or aryl of six to ten carbon atoms;
- R2: is C1 to C22 branched or straight chain alkyl or aryl of six to ten carbon atoms;
- R3: is C1 to C22 branched or straight chain alkyl or aryl of six to ten carbon atoms;
- R4: is C11 to C22 branched or straight chain alkyl or aryl of six to ten carbon atoms;
- q: is from 1 to about 2000;
- t: is from 1 to about 2000; the sum of q + t is from 2 to about 2000;
- R5: is C1 to C22 branched or straight chain alkyl or aryl of six to ten carbon atoms;
- R6: is C1 to C22 branched or straight chain alkyl or aryl of six to ten carbon atoms;
- R7: is C1 to C22 branched or straight chain alkyl or aryl of six to ten carbon atoms;
- R8: is C1 to C22 branched or straight chain alkyl or aryl of six to ten carbon atoms;
- a: is from 1 to about 100;
- b: is from 1 to about 100; the sum of a + b is from 2 to about 100; and
- x: is an integer from 1 to about 2000.

It will often be desirable to coat the pigment particles with, for example, one or more hydrous inorganic oxides, for example, hydrated silica or alumina, TiO₂, Fe-oxides, tin oxide, mica, zinc oxide, colorants like carmine or Prussian blue.

Examples of pigments include inorganic pigments, metal oxides and hydroxides, mica, organic pigments, pearlescent pigments, mineral silicates, porous materials, carbons, interference pigments, and the like.

Preferred pigment particles are selected from the group consisting of inorganic pigments, metal oxides and hydroxides, mica, organic pigments, pearlescent pigments, mineral silicates, porous materials, carbons, and interference pigments.

Examples of the inorganic pigments capable of being modified are ultramarine blue, Prussian blue, manganese violet, titanium-coated mica, bismuth oxychloride, iron oxides, iron hydroxide, titanium dioxide, titanium lower oxides, and chromium hydroxide.

Examples of the metal oxides and hydroxides capable of being modified are magnesium oxide, magnesium hydroxide, calcium oxide, calcium hydroxides, aluminum oxide, aluminum hydroxide, silica, iron oxides (α-Fe₂O₃, γ-Fe₂O₃, Fe₃O₄, FeO), iron hydroxides, titanium dioxide, titanium lower oxides, zirconium oxide, chromium oxides, chromium hydroxides, manganese oxides, cobalt oxides, nickel oxides, and zinc oxides. These oxides and hydroxides may be used alone or in any mixture thereof. Furthermore, composite oxides and composite hydroxides such as iron titanate, cobalt titanate, and cobalt aluminate can also be used in the present invention.

Composite materials comprising metal oxides or hydroxides coated on the core materials (e.g., titanium oxides coated mica, iron oxides coated nylon) can also be used in the present Invention.

Examples of the organic pigments capable of being modified with the silicone compounds of formula (SC1), SC(2), or (SC3) are C.I. 15850, C.I. 15850:1, C.I. 15585:1, C.I. 15630, C.I. 15880:1, C.I. 73360, C.I. 12085, C.I. 15865:2, C.I. 12075, C.I. 21110, C.I. 21095, and C.I. 11680, C.I. 74160 and zirconium, barium, or aluminum lakes of C.I. 45430, C.I. 45410, C.I. 45100, C.I. 17200, C.I. 45380, C.I. 45190, C.I. 14700, C.I. 15510, C.I. 19140, C.I. 15985, C.I. 45350, C.I. 47005, C.I. 42053, C.I. 42090.

C.I. means Colour Index as compiled by the by The Society of Dyers and Colourists and The American Association of Textile Chemists and Colourists.

The range of interesting organic pigments may additionally comprise monoazo, disazo, naphthol, dioxazone, azomethin, azocondensation, metal complex, nitro, perinone, quinoline, anthraquinone, benzimidozolone, isoindoline, isoindolinone, triarylmethane, quinacridone, hydroxyanthraquinone, aminoanthraquinone, anthrapyrimidine, indanthrone, flavanthrone, pyranthrone, anthantrone, isoviolanthrone, diketopyrrolopyrrole, carbazole, indigo or thiolndigo pigments.

Mixtures of the organic pigments may be used.

Examples of these pigments and also further pigments can be found in the monograph: W. Herbst, K. Hunger "Industrielle Organische Pigmente," 2.sup.nd edition, 1995, VCH Verlagsgesellschaft.

Preferred pigments are mono- or disazo pigments, preferably mono- or diarylides, or metal complexes, preferably a copper phthalocyanine pigment, or naphthol pigments, preferably β-naphthol or α,β-oxynaphthoic acid (BONA) pigments, or quinacridones or indanthrones.

More preferably, the following organic pigments are used inventively:
- Mono- or diarylide yellow pigments selected from C.I. Pigment Yellow 1, 2, 10, 12, 13, 14, 17, 61, 62, 63, 64, 65, 73, 74, 75, 83, 127, 168, 174, 176, 188 and 191;
- Monoarylide yellow pigments selected from C.I. Pigment Yellow 1, C.I.Pigment Yellow 2, C.I.Pigment Yellow 65, C.I.Pigment Yellow 73, C.I.Pigment Yellow 74 and C.I. Yellow 75;
- Diarylide yellow pigments selected from C.I.Pigment Yellow 12, C.I. Pigment Yellow 13, C.I. Pigment Yellow 14, C.I. Pigment Yellow 17 and C.I. Pigment Yellow 83;
- Disazo orange pigments selected from C.I. Pigment Orange 16 and C.I. Pigment Orange 34;
- Naphthol red pigments selected from C.I. Pigment Red 48:1, C.I. Pigment Red 48:2, C.I. Pigment Red 48:3, C.I. Pigment Red 48:4, C.I. Pigment Red 48:5, C.I. Pigment Red 49:1, C.I. Pigment Red 52:1, C.I. Pigment Red 52:2, C.I. Pigment Red 52:3, C.1 Pigment Red 53:1, C.I. Pigment Red 53:2, C.I. Pigment Red 53:3, C.I. Pigment Red 57:1, C.I. Pigment Red 57:2, C.I. Pigment Red 58:2, C.I. Pigment Red 58:4, C.I. Pigment Red 63:1 and C.I. Pigment Red 64:1;
- Quinacridone red pigments such as C.I. Pigment Red 202;
- Blue or green copper phthalocyanine pigments selected from C.I. Pigment Blue 15:1, C.I. Pigment Blue 15:2, C.I. Pigment Blue 15:3, C.I. Pigment Blue 15:4, C.I. Pigment Green 7 and C.I. Pigment Green 36;
- Blue indanthrone pigments such as C.I. Pigment Blue 60.

The surfaces of these organic pigments may be treated additionally with, for example, rosins.

Examples of pearlescent pigments (or nacreous pigments) are mica-titanium composite materials containing, as a titanium component, titanium dioxide, titanium lower oxides, titanium oxynitride, mica-iron oxide composite materials, bismuth oxychloride, and guanine. The mica-titanium composite materials may be mixed with colored pigments such as iron oxides, Prussian blue, chromium oxide, carbon black, and carmine. These pearlescent pigments may be used alone or in any mixture thereof.

The interference pigments are platelet particulates. The platelet particulates preferably have a thickness of no more than about 5 micrometers, more preferably no more than about 2 micrometers, still more preferably no more than about 1 micrometer. The platelet particulates preferably have a thickness of at least about 0.02 micrometers, more preferably at least about 0.05 micrometers, even more preferably at least about 0.1 micrometers, and still more preferably at least about 0.2 micrometers.

The particle size determines the opacity and luster. The particle size is determined by measuring the diameter thickness of the particulate material. The term "diameter" as used herein, means the largest distance across the major axis of the particulate material. Diameter can be determined by any suitable method known in the art, such as particle size analyzer Mastersizer 2000 manufactured by Malvern Instruments. The interference pigments preferably have an average diameter not greater than about 200 micrometers, more preferably not greater than 150 micrometers. The interference pigments preferably have a diameter of at least about 0.1 micrometer, more preferably at least about 1.0 micrometer, even more preferably at least about 2.0 micrometer, and still more preferably at least about 5.0 micrometer.

The interference pigments comprise a multilayer structure. The center of the particulates is a flat substrate with a refractive index (RI) normally below 1.8. A wide variety of particle substrates are useful herein. Nonlimiting examples are natural mica, synthetic mica, graphite, talc, kaolin, alumina flake, bismuth oxychloride, silica flake, glass flake, ceramics, titanium dioxide, CaSO₄, CaCO₃, BaSO₄, borosilicate and mixtures thereof, preferably mica, silica and alumina flakes.

A layer of thin film or a multiple layer of thin films are coated on the surface of a substrate described above. The thin films are made of highly refractive materials. The refractive index of these materials is normally above 1.8.

A wide variety of thin films are useful herein. Nonlimiting examples are TiO₂, Fe₂O₃, SnO₂, Cr₂O₃, ZnO, ZnS, SnO, ZrO₂, CaF₂, Al₂O₃, BiOCI, and mixtures thereof or in the form of separate layers, preferably TiO₂, Fe₂O₃, Cr₂O₃ and SnO₂.

For the multiple layer structures, the thin films can be consisted of all high refractive index materials or alternation of thin films with high and low RI materials with the high RI film as the top layer.

The interference pigment color is a function of the thickness of thin film, the thickness for a specific color may be different for different materials. For TiO₂, a layer of 40 nm to 60 nm or a whole number multiple thereof gives silver color, 60 nm to 80 nm yellow color, 80 nm to 100 nm red color, 100 nm to 130 nm blue color, 130 nm to 160 nm green color. In addition to the interference color, other transparent absorption pigments can be precipitated on top of or simultaneously with the TiO₂ layer. Common materials are red or black iron oxide, ferric ferrocyanide, chromium oxide or carmine. It is found that the color of the interference pigment in addition to its brightness has a significant influence on human perception of skin tone. In general, preferred colors are silver, gold, red, green and mixtures thereof.

Nonlimiting examples of the interference pigments useful herein include those supplied by Persperse, Inc. under the trade name PRESTIGE.RTM., FLONAC.RTM.; supplied by EMD Chemicals, Inc. under the trade name TIMIRON.RTM., COLORONA.RTM., DICHRO-NA.RTM. and XIRONA.RTM.; supplied by Engelhard Co. under the trade name FLAMENCO.RTM., TIMICA.RTM., DUOCHROME.RTM.; and supplied by Ciba under the trade name CALISHA.RTM.

The particulate organic UV-filters (b₁) according to the present invention are present in the micronized state. They may be prepared by any known process suitable for the preparation of microparticles, for example:wet-milling, wet-kneading, spray-drying from a suitable solvent, by expansion according to the RESS process (Rapid Expansion of Supercritical Solutions) of supercritical fluids (e.g. CO₂, by reprecipitation from suitable solvents, including supercritical fluids (GASR process = Gas Anti-Solvent Recrystallisation / PCA process = Precipitation with Compressed Anti-solvents).

As milling apparatus for the preparation of the sparingly soluble micronised organic compounds there may be used, for example, a jet mill, ball mill, vibratory mill or hammer mill, preferably a high-speed mixing mill. Even more preferable mills are modern ball mills; manufacturers of these types of mill are, for example, Netzsch (LMZ mill), Drais (DCP-Viscoflow or Cosmo), Bühler AG (centrifugal mills) or Bachhofer.

Examples of kneading apparatus for the preparation of the micronised organic UV absorbers are typical sigma-blade batch kneaders but also serial batch kneaders (IKA-Werke) or continuous kneaders (Continua from Werner und Pfleiderer).

The grinding of organic compounds used in the present invention is preferably carried out with a grinding aid.

Preferably, a dispersing agent is used as a low molecular weight grinding aid for all the above micronisation processes.

Preferred useful grinding aids for an aqueous dispersion are anionic surfactants with a HLB (Hydrophile-Lipophile Balance) value higher than 8, more preferably higher than 10.

Any conventionally usable anionic, non-ionic or amphoteric surfactants can be used as dispersing agents (component (b)). Such surfactant systems may comprise for example: carboxylic acids and their salts: alkaline soap of sodium, potassium and ammonium, metallic soap of calcium or magnesium, organic basis soap such as Lauric, myristic, palmitic, stearic and oleic acid etc., alkyl phosphates or phosphoric acid esters, acid phosphate, diethanolamine phosphate, potassium cetyl phosphate,. ethoxylated carboxylic acids or polyethyleneglycol esters, PEG-n acrylates, fatty alcohol polyglycolether such as laureth-n, myreth-n, ceteareth-n, steareth-n, oleth-n, fatty acid polyglycolether such as PEG-n stearate, PEG-n oleate, PEG-n cocoate, monoglycerides and polyol esters, C12-C22 fatty acid mono- and di-esters of addition products of from 1 to 100 mol of ethylene oxide with polyols,fatty acid and polyglycerol ester such as monostearate glycerol, diisostearoyl polyglyceryl-3-diisostearates, polyglyceryl-3-diisostearates, triglyceryl diisostearates, polyglyceryl-2-sesquiisostearates or polyglyceryl dimerates. mixtures of compounds from a plurality of those substance classes are also suitable, fatty acid polyglycolesters such as monostearate diethylene glycol, fatty acid and polyethylene glycol esters, fatty acid and saccharose esters such as sucro esters, glycerol and saccharose esters such as sucro glycerides. Sorbitol and sorbitan, sorbitan mono- and di-esters of saturated and unsaturated fatty acids having from 6 to 22 carbon atoms and ethylene oxide addition products, polysorbate-n series, sorbitan esters such as sesquiisostearate, sorbitan, PEG-(6)-isostearate sorbitan, PEG-(10)-sorbitan laurate, PEG-17- dioleate sorbitan, glucose derivatives, C8-C22 alkyl-mono and oligo-glycosides and ethoxylated analogues with glucose being preferred as the sugar component, O/W emulsifiers such as methyl gluceth-20 sesquistearate, sorbitan stearate/sucrose cocoate, methyl glucose sesquistearate, cetearyl alcohol/cetearyl glucoside, W/O emulsifiers such as methyl glucose dioleate/ methyl glucose isostearate. Sulfates and sulfonated derivatives, dialkylsulfosuccinates, dioctyl succinate, alkyl lauryl sulfonate, linear sulfonated parafins, sulfonated tetraproplyne sulfonate, sodium lauryl sulfates, amonium and ethanolamine lauryl sulfates, lauryl ether sulfates, sodium laureth sulfates [Texapon N70] or sodium myreth sulfates [Texapon K14S], sulfosuccinates, aceyl isothionates, alkanolamide sulfates, taurines, methyl taurines, imidazole sulfates, zwitterionic or amphoteric surfactants that carry at least one quaternary ammonium group and at least one carboxylate and/or sulfonate group in the molecule, zwitterionic surfactants that are especially suitable are betaines, such as N-alkyl-N,N-dimethylammonium glycinates, cocoalkyldimethylammonium glycinate, N-acylaminopropyl-N,N-dimethylammonium glycinates, cocoacylaminopropyldimethylammonium glycinate and 2-alkyl-3-carboxymethyl-3-hydroxyethylimidazolines each having from 8 to 18 carbon atoms in the alkyl or acyl group and also cocoacylaminoethylhydroxyethylcarboxymethylglycinate, N-alkylbetaine, N-alkylaminobetaines.

Examples of suitable mild surfactants as dispersing agents, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, α-olefin sulfonates, ethercarboxylic acids, alkyl oligoglucosides, fatty acid glucamides, alkylamidobetaines and/or protein fatty acid condensation products, the latter preferably being based on wheat proteins.

Non ionic surfactants such as PEG-6 beeswax (and) PEG-6 stearate (and) polyglyceryl2-isostearate [Apifac], glyceryl stearate (and) PEG-100 stearate. [Arlacel 165], PEG-5 glyceryl stearate [arlatone 983 S], sorbitan oleate (and) polyglyceryl-3 ricinoleate.[Arlacel 1689], sorbitan stearate and sucrose cocoate [arlatone 2121], glyceryl stearate and laureth-23 [Cerasynth 945], cetearyl alcohol and ceteth-20 [Cetomacrogol Wax], cetearyl alcohol and colysorbate 60 and PEG-150 and stearate-20[Polawax GP 200, Polawax NF], cetearyl alcohol and cetearyl polyglucoside [Emulgade PL 1618], cetearyl alcohol and ceteareth-20 [Emulgade 1000NI, Cosmowax], cetearyl alcohol and PEG-40 castor oil [Emulgade F Special], cetearyl alcohol and PEG-40 castor oil and sodium cetearyl sulfate [Emulgade F], stearyl alcohol and steareth-7 and steareth-10 [Emulgator E 2155], cetearyl alcohol and szeareth-7 and steareth-10 [Emulsifying wax U.S.N.F], glyceryl stearate and PEG-75 stearate [Gelot 64], propylene glycol ceteth-3 acetate .[Hetester PCS], propylene glycol isoceth-3 acetate [Hetester PHA], cetearyl alcohol and ceteth-12 and oleth-12 [Lanbritol Wax N 21], PEG -6 stearate and PEG-32 stearate [Tefose 1500], PEG-6 stearate and ceteth-20 and steareth-20 [Tefose 2000], PEG-6 stearate and ceteth-20 and glyceryl stearate and steareth-20 [Tefose 2561], glyceryl stearate and ceteareth-20 [Teginacid H, C, X].

Anionic emulsifiers such as PEG-2 stearate SE, glyceryl stearate SE [Monelgine, Cutina KD], propylene glycol stearate [Tegin P], cetearyl Alcohol and Sodium cetearyl sulfate [Lanette N, Cutina LE, Crodacol GP], cetearyl alcohol and sodium lauryl sulfate [Lanette W], trilaneth-4 phopshate and glycol stearate and PEG-2 stearate [Sedefos 75], glyceryl stearate and sodium lauryl Sulfate [Teginacid Special]. Cationic acid bases such as cetearyl alcohol and cetrimonium bromide.

Most preferred dispersing agents are sodium alkyl sulfates or sodium alkyl ether sulfates, such as sodium laureth sulfate [Texapon N70 from Cognis] or sodium myreth sulfate [Texapon K14 S from Cognis].

Useful solvents are water, brine, (poly-)ethylene glycol, glycerol or cosmetically acceptable oils. Other useful solvents are disclosed below in the sections entitled "Esters of fatty acids", "Natural and synthetic triglycerides, including glyceryl esters and derivatives", "Pearlescent waxes", "Hydrocarbon oils" and "Silicones or siloxanes".

The micronised particulate organic compounds so obtained usually have an average particle size from 0.02 to 2 micrometres, preferably from 0.03 to 1.5 micrometres and more especially from 0.05 to 1.0 micrometres.

The aqueous dispersion comprising a micronized UV absorber used in the present invention generally comprises
30 - 60, preferably 35 to 55 parts of the sparingly soluble organic micronized substance;
2 - 20, preferably 3 to 10 parts of the dispersing agent;
0.1 - 1 part, preferably 0.1 to 0.5 parts of a thickening agent (for example xanthan gum); and
20 - 67.9 parts of water;

The cosmetic formulations or pharmaceutical compositions according to the present invention can also comprise one or more than one further UV filter which are different from the particulate organic UV-filters (b₁); and the soluble organic UV filters (b₂) as listed in Table 1:

| Table 2: Suitable UV filter substances and adjuvants which can be additionally used with the UV absorbers according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts (Mexoryl SL) | 56039-58-8 |
| 12 | Methyl N, N, N-trimethyl-4-[(4, 7, 7 -trimethyl-3-oxobicyclo[2,2, 1]hept-2-ylidene)methyl]anilinium sulphate (Mexoryl SO) | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; Octocrylene | 6197-30-4 |
| 30 | 2- ethylhexyl 4- (dimethylamino)benzoate | 21245-02-3 |
| 31 | 2- ethylhexyl 4- methoxycinnamate; Octyl Methoxy Cinnamate | 5466-77-3 |
| 32 | 2- ethylhexyl salicylate | 118-60-5 |
| 34 | 4- aminobenzoic acid | 150-13-0 |
| 35 | Benzoic acid, 4-amino-, ethyl ester, polymer with oxirane | 113010-52-9 |
| 38 | 2- phenyl- 1H- benzimidazole- 5- sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 40 | Triethanolamine salicylate | 2174-16-5 |
| 41 | 3, 3'-(1,4-phenylenedimethylene)bis[7, 7-dimethyl- 2-oxobicyclo[2.2.1]heptane-1 methanesulfonic acid] (Cibafast H) | 90457-82-2 |
| 42 | Titanium dioxide (primary particle size 10 - 50 nm); for example T805 or Eusolex T-AVO, Eusolex T-2000, Titaniumdioxid VT 817 | 13463-67-7 |
| 44 | Zinc oxide (primary particle size 20-100 nm); for example Zinc oxide NDM, Zinc oxide Z-Cote HP1, Nanox Zinc oxide | 1314-13-2 |
| 47 | 1H-Benzimidazole-4,6-disulfonic acid, 2,2'-(1,4-phenylene)bis-, disodium salt | 180898-37-7 |
| 48 | Benzoic acid, 4,4'-[[6-[[4-[[(1,1-dimethylethyl)amino]carbonyl]-phenyl]amino]1,3,5-triazine-2,4-diyl]diimino]bis-, bis(2-ethylhexyl)-ester; diethylhexyl butamido triazone (Uvasorb HEB) | 154702-15-5 |
| 49 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| 50 | Dimethicodiethylbenzalmalonate; Polysilicone 15 (Parsol SLX) | 207574-74-1 |
| 51 | Benzenesulfonic acid, 3-(2H-benzotriazol-2-yl)-4-hydroxy-5-(1-methylpropyl)-, monosodium salt (Tinogard HS) | 92484-48-5 |
| 53 | 1- Dodecanami nium, N-[3-[[4-(dimethylamino)benzoyl]amino]propyl]-N,N-dimethyl-, salt with 4-methylbenzenesulfonic acid (1:1) (Escalol HP610) | 156679-41-3 |
| 54 | 1-Propanaminium, N, N, N-trimethyl-3-[(1-oxo-3-phenyl-2-propenyl)-amino]-, chloride | 177190-98-6 |
| 55 | 1H-Benzimidazole-4,6-disulfonic acid, 2,2'-(1,4-phenylene)bis- | 170864-82-1 |
| 56 | 1,3,5-Triazine, 2,4,6-tris(4-methoxyphenyl)- | 7753-12-0 |
| 57 | 1,3,5-Triazine, 2,4,6-tris[4-[(2-ethylhexyl)oxy]phenyl]- | 208114-14-1 |
| 58 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 59 | 2-Propenoic acid, 3-(1H-imidazol-4-yl)- | 104-98-3 |
| 60 | Benzoic acid, 2-hydroxy-, [4-(1-methylethyl)phenyl]methyl ester | 94134-93-7 |
| 61 | 1,2,3-Propanetriol, 1-(4-aminobenzoate) (Glyceryl PABA) | 136-44-7 |
| 62 | Benzeneacetic acid, 3,4-dimethoxy-a-oxo- | 4732-70-1 |
| 63 | 2-Propenoic acid, 2-cyano-3,3-diphenyl-, ethyl ester | 5232-99-5 |
| 64 | Anthralinic acid, p-menth-3-yl ester | 134-09-8 |
| 65 | 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7 |
| 69 | sterols (cholesterol, lanosterol, phytosterols), as described in WO0341675 | |
| 70 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 71 | alpha-lipoic-acid as described in DE 10229995 | |
| 72 | synthetic organic polymers as described in EP 1 371 358, [0033]-[0041] | |
| 73 | phyllosilicates as described in EP 1371357 [0034]-[0037] | |
| 74 | silica compounds as described in EP1371356, [0033]-[0041] | |
| 75 | inorganic particles as described in DE10138496 [0043]-[0055] | |
| 76 | latex particles as described in DE10138496 [0027]-[0040] | |
| 77 | 1H-Benzimidazole-4,6-disulfonic acid, 2,2'-(1,4-phenylene)bis-, disodium salt; Bisimidazylate (Neo Heliopan APC) | 180898-37-7 |
| 82 | Di-2-ethylhexyl-3,5-dimethoxy-4-hydroxy-benzalmalonate (Oxynex ST, EMD Chemicals, as described in US 20040247536) | |
| 83 | T-LiteTM MAX: Titanium Dioxide (and) Dimethoxydiphenylsilane (and) Triethoxycaprylylsilane Crosspolymer (and) Hydrated Silica (and) Aluminum Hydroxyde | |
| 84 | T-Lite SF: Titanium Dioxide (and) Aluminum Hydroxide (and) Dimethicone/Methicone Copolymer | |
| 85 | T-Lite SF-S: Titanium Dioxide (and) Hydrated Silica (and) | |
| | Dimethicone/ Methicone Copolymer (and) Aluminum Hydroxide | |
| 86 | Z-COTE^{®} MAX: Zinc Oxide (and) Diphenyl Capryl Methicone | |
| 87 | Z-COTE HP1: Zinc Oxide (and) Triethoxycaprylylsilane | |
| 88 | 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) | 288254-16-0 |

The cosmetic or pharmaceutical preparations can be prepared by physically mixing the UV absorber(s) with the adjuvant using customary methods, for example by simply stirring together the individual components, especially by making use of the dissolution properties of already known cosmetic UV absorbers, like octyl methoxy cinnamate, salicylic acid isooctyl ester, etc.

The cosmetic or pharmaceutical preparations may be, for example, creams, gels, lotions, alcoholic and aqueous/alcoholic solutions, emulsions, wax/fat compositions, stick preparations, powders or ointments. In addition to the above mentioned UV filters, the cosmetic or pharmaceutical preparations may contain further adjuvants as described below.

As water- and oil-containing emulsions (e.g. W/O, O/W, O/W/O and W/O/W emulsions or microemulsions) the preparations contain, for example, from 0.1 to 30 % by weight, preferably from 0.1 to 15 % by weight and especially from 0.5 to 10 % by weight, based on the total weight of the composition, of one or more UV absorbers, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the invention may also contain one or one more additional compounds as like fatty alcohols, esters of fatty acids, natural or synthetic triglycerides including glyceryl esters and derivatives, pearlescent waxes, hydrocarbon oils, silicones or siloxanes (organosubstituted polysiloxanes), fluorinated or perfluorinated oils, emulsifiers, djuvants and additives, super-fatting agents, surfactants, consistency regulators/thickeners and rheology modifiers, polymers, biogenic active ingredients, deodorising active ingredients, anti-dandruff agents, antioxidants, hydrotropic agents, preservatives, bacteria-inhibiting agents, perfume oils, colourants, polymeric beads or hollow spheres as SPF enhancers.

### Cosmetic or pharmaceutical preparations

Cosmetic or pharmaceutical formulations are contained in a wide variety of cosmetic preparations. There come into consideration, for example, especially the following preparations: skin-care preparations, bath preparations, cosmetic personal care preparations, foot-care preparations, light-protective preparations, skin-tanning preparations, depigmenting preparations, insect-repellents, deodorants, antiperspirants, preparations for cleansing and caring for blemished skin, hair-removal preparations in chemical form (depilation), shaving preparations, fragrance preparations, cosmetic hair-treatment preparations,

### Presentation forms

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hairstraightening preparations, liquid hair-setting preparations, hair foams and hairsprays. Of special interest are hair-washing preparations in the form of shampoos.

Other typical ingredients in such formulations are preservatives, bactericides and bacteriostatic agents, perfumes, dyes, pigments, thickening agents, moisturizing agents, humectants, fats, oils, waxes or other typical ingredients of cosmetic and personal care formulations such as alcohols, poly-alcohols, polymers, electrolytes, organic solvents, silicon derivatives, emollients, emulsifiers or emulsifying surfactants, surfactants, dispersing agents, antioxidants, anti-irritants and anti-inflammatory agents etc.

Preferably the particulates (a) are used which can easily be removed from textiles in customary laundering processes.

Other embodiments of the present invention are the use of the cosmetic formulation according to the present invention
- for minimizing and/or masking the whitening effect and/or the particular color of a sunscreen composition;
- for the visualization of a consistent application of a sunscreen formulation;
- for the protection against electromagnetic radiation in the range >380nm;
- for adjusting a natural skin-like color tone of the sunscreen formulation;
- for promoting an even skin tone; and
- for minimising fine lines and wrinkles.

The composition is used for preventing the damaging effect of UVA and blue light and provides an optimal protection against UVA-induced photoaging of human skin also in conjunction with excessive exposure to longer wave-length light and IR

The cosmetic compositions according to the present invention may also be used for preventing and treating undesired skin pigmentation; preventing and treating photoaging of human skin; inhibiting the degradation or condensations of endogenous vitamins, antioxidants and cellular modulators such as carotenoids, flavonoids or unsaturated lipids present in the skin; treating skin disorders caused by sunlight such as pigment spots, actinic keratosis and actinic dermatitis and solar urticaria; protecting against UVA1-induced expression of matrix-degrading enzymes such as metallo-proteinases or elastases, e.g. metallo-proteinase-1 or neutrophil elastase or of cytokines such as interleukin-1 or interleukin-6; preventing the generation of reactive oxygen species such as by photosensitizing molecules, via UV-induced inflammatory reactions or via impaiment of mitochondria function; protecting UV-A-sensitive pharmaceuticals and cosmetics.

The compositions according to the present invention may constitute a skin care product, in particular for the face, neck, contours of the eye or the body, or a skin makeup product such as a tinting product, an eye shadow, a blusher, an eyeliner, a concealer, a body makeup product, a daily care product, a sun protection product or a skin cleansing product.

The cosmetic preparation according to the invention is distinguished by excellent protection of human skin against the damaging effect of sunlight.

### Examples

### Preparation of Sunscreen Formulation

Examples 1, 2 and 4 show advantage over Examples 3 and 5 which do not contain color pigments.

| INCI | | | | | | | Ex. 1 | | Ex. 2 | | Ex. 3 | |
|---|---|---|---|---|---|---|---|---|---|---|---|---|
| Part A | | | | | | | | | | | | |
| Glyceryl Stearate (and) PEG 100 Stearate | | | | | | | 2.00 | | 2.00 | | 2.00 | |
| Butylene Glycol Cocoate | | | | | | | 5.00 | | 5.00 | | 5.00 | |
| Isopropyl Palmitate | | | | | | | 4.00 | | 4.00 | | 4.00 | |
| Trioctanion | | | | | | | 4.00 | | 4.00 | | 4.00 | |
| TiO₂ (and) Stearic Acid (and) Aluminium Hydroxide | | | | | | | 3.20 | | 3.20 | | 3.20 | |
| Part B | | | | | | | | | | | | |
| Water | | | | | | | | | | | | |
| Tinoderm SGP (Ciba) | | | | | | | 3.00 | | 3.00 | | 3.00 | |
| Disodium EDTA | | | | | | | 0.20 | | 0.20 | | 0.20 | |
| Xanthan Gum | | | | | | | 0.20 | | 0.20 | | 0.20 | |
| Potassium cetyl Phosphate | | | | | | | 1.50 | | 1.50 | | 1.50 | |
| Part B2 | | | | | | | | | | | | |
| Calisha Contemporary jet black (Iron oxide) | | | | | | | 0.01 | | 0.005 | | - | |
| Calisha Contemporary medium red (iron oxide) | | | | | | | 0.1 | | 0.025 | | - | |
| Calisha Contemporary yellow (iron oxide) | | | | | | | 0.55 | | 0.27 | | - | |
| Part C | | | | | | | | | | | | |
| Phenyl Trimethicone | | | | | | | 2.00 | | 2.00 | | 2.00 | |
| Cyclomethicone | | | | | | | 4.00 | | 4.00 | | 4.00 | |
| Dimethicone (and) Dimethicone/Vinyl Dimethicone Crosspolymer | | | | | | | 3.00 | | 3.00 | | 3.00 | |
| Part D | | | | | | | | | | | | |
| Mica (and) Titanium Dioxide (and) Red iron oxide (and) Cetyl Dimethicone | | | | | | | 0.30 | | | | | |
| Micronized Methylene Bis-Benzotriazolyl Tetramethylbutylphenol | | | | | | | 4.00 | | 5.00 | | 3.00 | |
| Micronized 1,3,5-Triazine, 2,4,6-tris([1,1'-biphenyl]-4-yl)- (CAS Regn. 31274-51-8) | | | | | | | 5.00 | | 5.00 | | 5.00 | |
| Phenoxyethanol (and) Methylparaben (and) Ethylparaben (and) Butylparaben (and) Propylparaben Isobutylparaben | | | | | | | 1.00 | | | | | |
| Tinoderm E | | | | | | | | | | | 0.50 | |
| Water | | | | | | | ad 100 | | ad 100 | | ad 100 | |
| | | | | | | | | | | | | |

| | INCI | | | | | | | | Ex. 4 | | Ex. 5 | |
|---|---|---|---|---|---|---|---|---|---|---|---|---|
| Phase A | Glyceryl Stearate (and) PEG 100 Stearate | | | | | | | | 5.00 | | 5.00 | |
| | Phenethyl Benzoate | | | | | | | | 6.00 | | 6.00 | |
| | Isopropyl Palmitate | | | | | | | | 5.00 | | 5.00 | |
| | Triheptanoin | | | | | | | | 3.00 | | 3.00 | |

| | INCI | | | | | | | Ex. 4 | | | Ex. 5 | |
|---|---|---|---|---|---|---|---|---|---|---|---|---|
| | Bis-Ethylhexyloxyphenol Methoxyphenyl Triazine | | | | | | | 2.00 | | | 2.00 | |
| | Ethylhexyl Triazone | | | | | | | 1.50 | | | 1.50 | |
| Phase B | Water | | | | | | | | | | | |
| | Butylene Glycol | | | | | | | 2.00 | | | 2.00 | |
| | Sodium EDTA | | | | | | | 0.20 | | | 0.20 | |
| Phase B2 | Calisha Contemporary jet black (Iron oxide) | | | | | | | 0.01 | | | | |
| | Calisha Contemporary medium red (iron oxide) | | | | | | | 0.08 | | | | |
| | Calisha Contemporary yellow (iron oxide) | | | | | | | 0.55 | | | | |
| Phase C | Sodium Acrylates Copolymer (and) Mineral Oil (and) PPG-1 Trideceth-6 | | | | | | | 1.20 | | | 1.20 | |
| Phase D | Cyclomethicone | | | | | | | 3.00 | | | 3.00 | |
| | Cyclomethicone | | | | | | | 4.00 | | | 4.00 | |
| Phase E | Methylene Bis-Benzotriazolyl Tetramethylbutylphenol | | | | | | | 14.00 | | | 14.00 | |
| | Methylparaben (and) Ethylparaben (and) Butylparaben (and) Propylparaben | | | | | | | 1.00 | | | 1.00 | |
| | Water | | | | | | | ad 100 | | | ad 100 | |

### Manufacturing process:

Heat up part A and B to 75°C.
Add Amphisol K into B and stirr for 5 more minutes, then Add part B2.
Add part A into part B, then homogenize with an Ultra Turrax for 1 min pos 2/200g
Add part C by 60°C, and turrax again
Add part D below 50°C

### B. Application Examples

| Example B1: O/W Emulsion (1) | | | | |
|---|---|---|---|---|
| Part | INCI | A | B | C |
| A | Glyceryl Stearate (and) PEG 100 Stearate | 2.00 | 2.00 | 2.00 |
| | Butylene Glycol Cocoate | 5.00 | 5.00 | 5.00 |
| | Isopropyl Palmitate | 4.00 | 4.00 | 4.00 |
| | Triethylhexanoin | 4.00 | 4.00 | 4.00 |
| | TIO2 (and) Stearic Acid (and) Aluminum Hydroxide | 3.20 | 3.20 | 3.20 |
| B | | | | |
| | Pentylene Glycol (and) Sclerotium Gum | 3.00 | 3.00 | 3.00 |
| | Disodium EDTA | 0.20 | 0.20 | 0.20 |
| | Xanthan Gum | 0.20 | 0.24 | 0.24 |
| | Potassium cetyl Phosphate | 1.50 | 1.50 | 1.50 |
| B 2 | Iron Oxides (CALISHA Contemprorary Jet Black Oxide) | 0.01 | 0.005 | 0.005 |
| | Iron Oxides (CALISHA Contemporary Medium Red Oxide) | 0.10 | 0.025 | 0.025 |
| | Iron Oxides (CALISHA Contemporary Yellow Oxide) | 0.55 | 0.27 | 0.27 |
| C | Phenyl Trimethicone | 2.00 | 2.00 | 2.00 |
| | Cyclomethicone | 4.00 | 4.00 | 4.00 |
| | Dimethicone (and) Dimethicone/Vinyl Dimethicone Crosspolymer | 3.00 | 3.00 | 3.00 |
| D | Micronized Methylene Bis-Benzotriazolyl Tetramethyl-butylphenol | 4.00 | 4.00 | 4.00 |
| | Micronized 1,3,5-Triazine, 2,4,6-tris([1,1'-biphenyl]-4-yl)-(CAS Reqn. 31274-51-8) | 5.00 | 5.00 | 5.00 |
| | Micronized Methanone, 1,1'-(1,4-piperazinediyl)bis[1-[2-[4-(diethylamino)-2-hydroxybenzoyl]phenyl]-(CAS Regn. 919803-06-8) | 1.00 | 2.00 | 4.00 |
| | Mica (and) Titanium Dioxide (and)Tin oxide | 0.30 | | 0.30 |
| | Phenoxyethanol (and) Methylparaben (and) Ethylparaben (and) Butylparaben (and) Propylparaben (and) Isobutylparaben | 1.00 | 1.00 | 1.00 |
| | Aqua (and) Tocopheryl Acetate (and) Caprylic/Capric Triglyceride (and) Polysorbate 80 (and) Lecithin | | | 0.50 |
| | Water | ad 100 | ad 100 | ad 100 |

| Example B2: O/W Emulsion (2) | | | | | | |
|---|---|---|---|---|---|---|
| INCI | A | B | C | D | E | F |
| Glyceryl Stearate (and) PEG 100 Stearate | 5.00 | 5.00 | 5.00 | 5.00 | 5.00 | 5.00 |
| Phenethyl Benzoate | 6.00 | 6.00 | 6.00 | 6.00 | 6.00 | 6.00 |
| Isopropyl Palmitate | 5.00 | 5.00 | 5.00 | 5.00 | 5.00 | 5.00 |
| Triethylhexanoin | 3.00 | 3.00 | 3.00 | 3.00 | 3.00 | 3.00 |
| Bis-Ethylhexyloxyphenol Methoxyphenyl Triazine | 2.00 | 2.00 | 2.00 | 2.00 | 2.00 | 2.00 |
| Ethylhexyl Triazone | 1.50 | 1.50 | 1.50 | 1.50 | 1.50 | 1.50 |
| Butylene Glycol | 2.00 | 2.00 | 2.00 | 2.00 | 2.00 | 2.00 |
| Disodium EDTA | 0.20 | 0.20 | 0.20 | 0.20 | 0.20 | 0.20 |
| Iron Oxides (CALISHA Contemprorary Jet Black Oxide) | 0.01 | 0.02 | 0.01 | 0.01 | 0.01 | 0.01 |
| Iron Oxides (CALISHA Contemporary Medium Red Oxide) | 0.08 | 0.5 | 0.08 | 0.08 | 0.08 | 0.08 |
| Iron Oxides (CALISHA Contemporary Yellow Oxide | 0.55 | 0.5 | 0.55 | 0.55 | 0.55 | 0.55 |
| Sodium Acrylates Copolymer (and) Mineral Oil (and) PPG-1 Trideceth-6 | 1.20 | 1.20 | 1.20 | 1.20 | 1.20 | 1.20 |
| Cyclomethicone | 4.00 | 4.00 | 4.00 | 4.00 | 4.00 | 4.00 |
| Micronized Methylene Bis-Benzotriazolyl Tetramethyl butyl phenol | 4.00 | 4.00 | 4.00 | 1.00 | 2.00 | 2.00 |
| Butyl Methoxydibenzoymethane | 0.5 | | 4.00 | 4.00 | | 0.5 |
| Ethylhexyl Methoxycinnamate | 0.5 | | 3.00 | 3.00 | | 0.5 |
| Octocrylene | 0.5 | | 4.00 | 4.00 | | 0.5 |
| Propanedinitrile, 2-[3-[bis(2-ethylhexyl)-amino]-2-cyclohexen-1-ylidene]- (CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) (CAS Reqn 288254-16-0) | | | | 2.00 | | 0.5 |
| Acetic acid, 2-cyano-2-[5,5-dimethyl-3-[(1-methyl propyl)amino]-2-cyclohexen-1-ylidene]-, 2-ethylhexyl ester (CAS Regn. 923271-35-6) | | 2.00 | | | 1.00 | 0.5 |
| Uvinul A plus (CAS Regn. 302776-68-7) | 0.5 | 0.5 | 2.00 | | | 2.00 |
| Micronized 1,3,5-Triazine, 2,4,6-tris([1,1'-biphenyl]-4-yl)- (CAS Regn. 31274-51-8) | 2.00 | 2.00 | 2.00 | 2.00 | 2.00 | 2.00 |
| 2,4-Pentadienoic acid, 5-(diethylamino)-2-(phenylsulfonyl)-, octyl ester (CAS Regn. 98835-90-6) | 2.00 | | 0.5 | 0.5 | 0.5 | 0.5 |
| Micronized Methanone, 1,1'-(1,4-piperazinediyl)bis[1-[2-[4-(diethylamino)-2-hydroxybenzoyl]phenyl]- (CAS Regn. 919803-06-8) | 2.00 | 2.00 | 2.00 | 2.00 | | 2.00 |
| Mica (and) Titanium Dioxide (and)Tin oxide | 1.00 | 1.00 | 1.00 | 1.00 | 1.00 | 1.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 | 1.00 |
| Water | ad 100 | ad 100 | ad 100 | ad 100 | ad 100 | ad 100 |

| Example B3: O/W Emulsion (3) | | | | | | |
|---|---|---|---|---|---|---|
| INCI | A | B | C | D | E | F |
| Tribehenin PEG-20 esters | 5.00 | 5.00 | 5.00 | 5.00 | 5.00 | 5.00 |
| Stearyl Alcohol | 0.50 | 0.50 | 0.50 | 0.50 | 0.50 | 0.50 |
| Dibutyl Adipate | 5.00 | 5.00 | 5.00 | 5.00 | 5.00 | 5.00 |
| PPG-2 Myristyl Ether Propionate | 5.00 | 5.00 | 5.00 | 5.00 | 5.00 | 5.00 |
| Triheptanoin | 3.00 | 3.00 | 3.00 | 3.00 | 3.00 | 3.00 |
| Ethylhexyl Salicylate | 5.00 | 5.00 | 5.00 | 5.00 | 5.00 | 5.00 |
| Butyl Methoxydibenzoymethane | 3.00 | 3.00 | 3.00 | 3.00 | 3.00 | 3.00 |
| Bis-Ethylhexyloxyphenol Methoxyphenyl Triazine | 3.00 | 3.00 | 3.00 | 3.00 | 3.00 | 3.00 |
| Ethylhexyl Triazone | 3.00 | 3.00 | 3.00 | 3.00 | 3.00 | 3.00 |
| Micronized Methylene Bis-Benzotriazolyl Tetramethylbutylphenol | 2.00 | 2.00 | 2.00 | 2.00 | 2.00 | 2.00 |
| Butyl Methoxydibenzoymethane | | 4.00 | | 0.5 | 0.5 | 0.5 |
| Bis-Ethylhexyloxyphenol Methoxyphenyl Triazine | 1.00 | 1.00 | 1.00 | 1.00 | 1.00 | 1.00 |
| Ethylhexyl Triazone | 0.5 | 0.5 | 0.5 | 0.5 | 0.5 | 0.5 |
| Ethylhexyl Methoxycinnamate | | 4.00 | | | | |
| Octocrylene | | 4.00 | | 0.5 | 0.5 | 0.5 |
| Propanedinitrile, 2-[3-[bis(2-ethylhexyl)amino]-2-cyclohexen-1-ylidene]-(CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]-phenyl]-N6-(2-ethylhexyl)- (Uvasorb K2A) (CAS Reqn 288254-16-0) | 2.00 | | 0.5 | | 1.0 | |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | | | 2.00 | | | |
| Uvinul A plus (CAS Regn. 302776-68-7) | | | | 2.00 | | |
| Micronized 1,3,5-Triazine, 2,4,6-tris-([1,1'-biphenyl]-4-yl)- (CAS Regn. 31274-51-8) | 2.00 | 5.00 | 2.00 | 2.00 | 2.00 | 4.00 |
| 2,4-Pentadienoic acid, 5-(diethylamino)-2-(phenylsulfonyl)-, octyl ester (CAS Reqn. 98835-90-6) | | 2.00 | | | | |
| Micronized Methanone, 1,1'-(1,4-pipe-razinediyl)bis[1-[2-[4-(diethylamino)-2-hyd roxybenzoyl]phenyl]-(CAS Reqn. 919803-06-8) | 2.00 | 2.00 | 5.00 | 4.00 | 2.00 | 2.00 |
| Sodium Polyacrylate | | 0.60 | 0.60 | 0.60 | 0.60 | 0.60 |
| Disodium EDTA | 0.20 | 0.20 | 0.20 | 0.20 | 0.20 | 0.20 |
| Xanthan Gum | 0.50 | | 0.50 | 0.50 | 0.50 | 0.50 |
| Iron Oxides (CALISHA Contemprorary Black Oxide) | 0.020 | 0.020 | 0.020 | 0.020 | 0.020 | 0.020 |
| Iron Oxides (CALISHA Contemporary Salmon Red) | 0.08 | | 0.08 | 0.08 | 0.08 | 0.08 |
| Iron Oxides (CALISHA Contemporary Sun Yellow) | 0.55 | | 0.55 | 0.55 | 0.55 | 0.55 |
| Cyclomethicone | 5.00 | 5.00 | 5.00 | 5.00 | 5.00 | 5.00 |
| Mica (and) Titanium Dioxide (and) Iron Oxides (and) Cetyl Dimethicone | 0.50 | 0.50 | 0.50 | 0.50 | 0.50 | 0.50 |
| Mica (and) Titanium Dioxide (and) Cetyl Dimethicone (and) Iron Oxides (and) Carmine | 0.50 | 0.50 | 0.50 | 0.50 | 0.50 | 0.50 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 | 1.00 |
| Water | ad 100 | ad 100 | ad 100 | ad 100 | ad 100 | ad 100 |

| Example B4: W/O Emulsion (1) | | | | | |
|---|---|---|---|---|---|
| INCI | A | B | C | D | E |
| Polyglyceryl -4 Isostearate (and) Cetyl PEG/PPG-10/1 Dimethicone (and) Hexyl Laurate | 4.50 | 4.50 | 4.50 | 4.50 | 4.50 |
| Cetyl PEG/PPG-10/1 Dimethicone | 2.20 | 2.20 | 2.20 | 2.20 | 2.20 |
| Sorbitan Sesquioleate | 1.00 | 1.00 | 1.00 | 1.00 | 1.00 |
| Cera Alba | 1.80 | 1.80 | 1.80 | 1.80 | 1.80 |
| Hydrogenated Vegetable Oil | 2.00 | 2.00 | 2.00 | 2.00 | 2.00 |
| Phenethyl Benzoate | 2.00 | 2.00 | 2.00 | 2.00 | 2.00 |
| Bis-Ethylhexyloxyphenol Methoxyphenyl Triazine | 1.00 | 1.00 | 3.00 | 1.00 | 1.00 |
| Ethylhexyl Methoxycinnamate | 5.00 | 5.00 | 5.00 | 10.00 | 5.00 |
| Titanium Dioxide (and) Silica (and) Dimethicone | 5.00 | 5.00 | 5.00 | 5.00 | 5.00 |
| Iron Oxides (and) Cetyl Dimethicone | 0.13 | 0.13 | 0.13 | 0.13 | 0.13 |
| Iron Oxides (and) Cetyl Dimethicone | 0.60 | 0.60 | 0.60 | 0.60 | 0.60 |
| Iron Oxides (and) Cetyl Dimethicone | 0.20 | 0.20 | 0.20 | 0.20 | 0.20 |
| Cyclomethicone (and) Disteardimonium Hectorite (and) Propylene Carbonate | 5.00 | 5.00 | 5.00 | 5.00 | 5.00 |
| Cyclopentasiloxane | 6.00 | 6.00 | 6.00 | 6.00 | 6.00 |
| Ethyl Trisiloxane | 8.00 | 8.00 | 8.00 | 8.00 | 8.00 |
| Dimethicone | 6.00 | 6.00 | 6.00 | 6.00 | 6.00 |
| Cyclopentasiloxane (and) C30-45 Alkyl Cetearyl Dimethicone Crosspolymer (and) PEG/PPG-20/23 Dimethicone | 1.00 | 1.00 | 1.00 | 1.00 | 1.00 |
| Water | ad 100 | ad 100 | ad 100 | ad 100 | ad 100 |
| Sodium Chloride | 1.00 | 1.00 | 1.00 | 1.00 | 1.00 |
| Pentylene Glycol (and) Scerotium Gum | 3.00 | 3.00 | 3.00 | 3.00 | 3.00 |
| Aqua (and) Retinyl Palmitate (and) Capryic/Capric Triglyceride (and) Polysorbate 80 (and) Lecithin (and) Benzalkonium Chloride | 3.00 | 3.00 | 3.00 | 3.00 | 3.00 |
| Butylen Glycol | 3.00 | 3.00 | 3.00 | 3.00 | 3.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 |
| Aluminum Starch Octenylsuccinate | 2.00 | 2.00 | 2.00 | 2.00 | 2.00 |
| Talc | 2.00 | 2.00 | 2.00 | 2.00 | 2.00 |
| Silica | 5.00 | 5.00 | 5.00 | 5.00 | 5.00 |
| Mica (and) Iron Oxides (and) Cetyl Dimethicone | 2.00 | 2.00 | 2.00 | 2.00 | 2.00 |
| Mica (and) Titanium Dioxide (and) Iron Oxides (and) Cetyl Dimethicone | | 1 | | 0.5 | |
| Micronized Methylene Bis-Benzotriazolyl Tetramethylbutylphenol | 0.5 | 6.00 | 4.00 | 2.00 | 1.00 |
| Butyl Methoxydibenzoymethane | 5.00 | 5.00 | 5.00 | 5.00 | 5.00 |
| Ethylhexyl Triazone | 0.60 | 0.60 | 0.60 | 0.60 | 0.60 |
| Octocrylene | 4.00 | 0.5 | 0.5 | 0.5 | 0.5 |
| Propanedinitrile, 2-[3-[bis(2-ethylhexyl)amino]-2-cyclohexen-1-ylidene]- (CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) (CAS Regn 288254-16-0) | 2.00 | 1.00 | 5.00 | 2.00 | 1.00 |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | 6.00 | 6.00 | 6.00 | 6.00 | 6.00 |
| Uvinul A plus (CAS Regn. 302776-68-7) | 8.00 | 8.00 | 8.00 | 8.00 | 8.00 |
| Micronized 1,3,5-Triazine, 2,4,6-tris([1,1'-biphenyl]-4-yl)- (CAS Regn. 31274-51-8) | 6.00 | 6.00 | 6.00 | 6.00 | 6.00 |
| 2,4-Pentadienoic acid, 5-(diethylamino)-2-(phenylsulfonyl)-, octyl ester (CAS Regn. 98835-90-6) | 1.00 | 1.00 | 1.00 | 1.00 | 1.00 |
| Micronized Methanone, 1,1'-(1,4-piperazine-diyl)bis[1-[2-[4-(diethylamino)-2-hydroxybenzoyl]-phenyl]- (CAS Regn. 919803-06-8) | 1.00 | 3.00 | 2.00 | 4.00 | 0.5 |

| Example B5: W/O Emulsion (2) | | | | | |
|---|---|---|---|---|---|
| INCI | A | B | C | D | E |
| PEG-30 Dipolyhydroxystearate | 3.00 | 3.00 | 3.00 | 3.00 | 3.00 |
| Butylen Glycol Cocoate | 2.00 | 2.00 | 2.00 | 2.00 | 2.00 |
| Isodecyl neopentanoate | 4.00 | 4.00 | 4.00 | 4.00 | 4.00 |
| Isopropyl Isosterate | 3.00 | 3.00 | 3.00 | 3.00 | 3.00 |
| Beeswax | 1.00 | 1.00 | 1.00 | 1.00 | 1.00 |
| Hydrogenated Castor Oil | 0.80 | 0.80 | 0.80 | 0.80 | 0.80 |
| Ethylhexyl Methoxycinnamate | 5.00 | 5.00 | 5.00 | 5.00 | 5.00 |
| Iron Oxides (and) Cetyl Dimethicone (Calisha Contemporary Black Oxide OD) | 0.10 | 0.10 | 0.10 | 0.10 | 0.10 |
| Iron Oxides (and) Cetyl Dimethicone (Calisha Contemporary Yellow Oxide OD) | 0.57 | 0.57 | 0.57 | 0.57 | 0.57 |
| Iron Oxides (and) Cetyl Dimethicone (Calisha Contemporary Medium Red Oxide OD) | 0.13 | 0.13 | 0.13 | 0.13 | 0.13 |
| Caprylyl Methicone | 2.50 | 2.50 | 2.50 | 2.50 | 2.50 |
| Water | ad 100 | ad 100 | ad 100 | ad 100 | ad 100 |
| Sodium Chloride | 1.00 | 1.00 | 1.00 | 1.00 | 1.00 |
| Pentylene Glycol (and) Scerotium Gum | 3.00 | 3.00 | 3.00 | 3.00 | 3.00 |
| Aqua (and) Retinyl Palmitate (and) Caprylic/Capric Triglyceride (and) Polysorbate 80 (and) Lecithin (and) Benzalkonium Chloride | 3.00 | 3.00 | 3.00 | 3.00 | 3.00 |
| Butylen Glycol | 5.00 | 5.00 | 5.00 | 5.00 | 5.00 |
| Polysorbate 80 | 0.25 | 0.25 | 0.25 | 0.25 | 0.25 |
| Caprylyl Methicone | 5.50 | 5.50 | 5.50 | 5.50 | 5.50 |
| Dimethicone | 7.00 | 7.00 | 7.00 | 7.00 | 7.00 |
| Micronized Methylene Bis-Benzotriazolyl Tetramethylbutylphenol | 3.00 | 6.00 | 3.00 | 6.00 | 2.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 |
| Silica | 3.00 | 3.00 | 3.00 | 3.00 | 3.00 |
| Nylon-12 | 6.00 | 6.00 | 6.00 | 6.00 | 6.00 |
| Bis-Ethylhexyloxyphenol Methoxyphenyl Triazine | 1.00 | 1.00 | 1.00 | 1.00 | 1.00 |
| Butyl Methoxydibenzoymethane | 5.00 | 5.00 | 5.00 | 5.00 | 5.00 |
| Bis-Ethylhexyloxyphenol Methoxyphenyl Triazine | 0.13 | 0.13 | 0.13 | 0.13 | 0.13 |
| Ethylhexyl Triazone | 0.60 | 0.60 | 0.60 | 0.60 | 0.60 |
| Octocrylene | 4.00 | 0.5 | 0.5 | 0.5 | 0.5 |
| Propanedinitrile, 2-[3-[bis(2-ethylhexyl)amino]-2-cyclohexen-1-ylidene]- (CAS Regn. 923271-36-7) or merocyanine derivative of formula (14) or 1,3,5-Triazine-2,4,6-triamine, N2,N4-bis[4-[5-(1,1-dimethyl propyl)-2-benzoxazolyl]phenyl]-N6-(2-ethylhexyl)- (Uvasorb K2A) (CAS Regn 288254-16-0 | 2.00 | 1.00 | 5.00 | 2.00 | 1.00 |
| Acetic acid, 2-cyano-2-[5,5-dimethyl-3-[(1-methyl propyl)amino]-2-cyclohexen-1-ylidene]-, 2-ethylhexyl ester (CAS Regn. 923271-35-6) | 6.00 | 6.00 | 6.00 | 6.00 | 6.00 |
| Uvinul A plus (CAS Regn. 302776-68-7) | 8.00 | 8.00 | 8.00 | 8.00 | 8.00 |
| Micronized 1,3,5-Triazine, 2,4,6-tris([1,1'-biphenyl]-4-yl)- (CAS Regn. 31274-51-8) | 6.00 | 6.00 | 6.00 | 6.00 | 6.00 |
| 2,4-Pentadienoic acid, 5-(diethylamino)-2-(phenylsulfonyl)-, octyl ester (CAS Regn. 98835-90-6) | 1.00 | 1.00 | 1.00 | 1.00 | 1.00 |
| Micronized Methanone, 1,1'-(1,4-piperazinediyl)bis[1-[2-[4-(diethylamino)-2-hydroxybenzoyl]phenyl]- (CAS Regn. 919803-06-8) | 1.00 | 3.00 | 2.00 | 4.00 | 0.5 |
| (Manganese modified) Titanium Dioxide | | 4.00 | | 1.00 | |
| Diethylhexyl Syringylidene Malonate | 2.00 | 1.00 | 3.00 | 4.00 | 0.5 |
| Propanedioic acid, 2-[(4-hydroxy-3,5-dimethoxyphenyl)methyl]-, 1,3-bis(2-ethylhexyl) ester; (CAS Reqn. 872182-46-2) | 1.00 | | 1.00 | | 5.00 |

## Claims

1. Non-therapeutic use of a cosmetic formulation comprising
(a) particulates having an absorption in the range of 400 to 800nm selected from inorganic colour pigments selected from iron (III) oxide
; and
(b) particulate organic UV-filter of formula 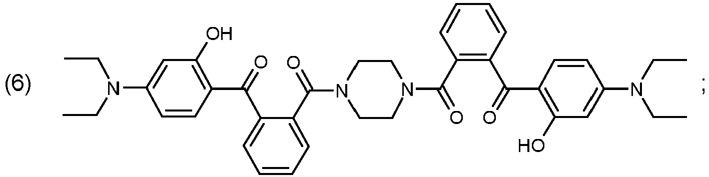 for minimizing and/or masking the whitening effect of a sunscreen composition.

2. Use according to claim 1, comprising
0.01 to 10 % b.w of particulates (a); and
0.1 to 40 % b.w of UV filters (b).

3. Use according to any of claims 1 or 2, wherein iron (III) oxide is selected from red iron oxide, black iron oxide, yellow iron oxide and mixtures thereof.

## Patentansprüche

1. Nichttherapeutische Verwendung einer kosmetischen Formulierung, umfassend
(a) Teilchen mit einer Absorption im Bereich von 400 bis 800 nm, ausgewählt aus anorganischen Farbpigmenten, ausgewählt aus Eisen(III)-oxid; und
(b) den teilchenförmigen organischen UV-Filter der Formel 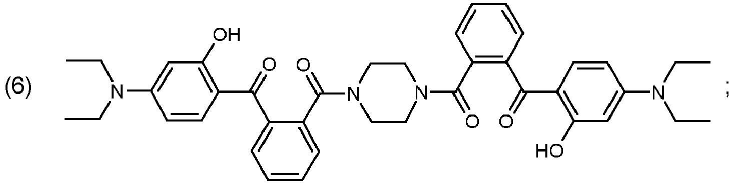 zum Minimieren und/oder Maskieren des Bleicheffekts einer Sonnenschutzzusammensetzung.

2. Verwendung nach Anspruch 1, umfassend 0,01 bis 10 Gew.-% Teilchen (a) und
0,1 bis 40 Gew.-% UV-Filter (b).

3. Verwendung nach Anspruch 1 oder 2, wobei das Eisen(III)-oxid aus Eisenrotoxid, Eisenschwarzoxid, Eisengelboxid und Mischungen davon ausgewählt ist.

## Revendications

1. Utilisation non thérapeutique d'une formulation cosmétique comprenant
(a) des particules possédant une absorption dans la plage de 400 à 800 nm choisies parmi des pigments de couleur inorganiques choisis parmi de l'oxyde de fer (III) ; et
(b) un filtre UV organique particulaire de formule 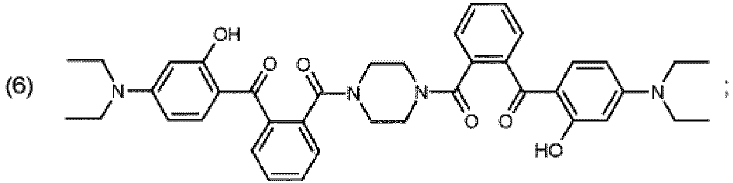 pour la minimisation et/ou le masquage de l'effet blanchissant d'une composition d'écran solaire.

2. Utilisation selon la revendication 1, comprenant 0,01 à 10 % en poids de particules (a) ; et
0,1 à 40 % en poids de filtres UV (b).

3. Utilisation selon l'une quelconque des revendications 1 et 2, l'oxyde de fer (III) étant choisi parmi l'oxyde de fer rouge, l'oxyde de fer noir, l'oxyde de fer jaune et des mélanges correspondants.
